# EUROPEAN PATENT SPECIFICATION

(11) **EP 0 618 220 B1**
(45) Date of publication and mention of the grant of the patent: **13.03.1996**
(21) Application number: 94105833.1
(22) Date of filing: 26.07.1990
(51) Int. Cl.: C07K 1/00, C12P 21/02, C12N 15/24

(54) **Method for purifying interleukin-4**
Verfahren zur Reinigung von Interleukin-4
Procédé de purification de l'interleukine-4

(30) Priority: 28.07.1989 US 386937
(43) Date of publication of application: 05.10.1994
(62) Divisional of application: 90308198.2
(73) Proprietor: SCHERING CORPORATION, Kenilworth New Jersey 07033 (US)
(72) Inventor: Bonnem, Eric, Plainfield, New Jersey 07062 (US); Sullivan, Lee, Edison, New Jersey 08837 (US); Grace, Michael, Hamilton Township, New Jersey 08620 (US); Bober, Loretta, Linden, New Jersey 07039 (US); Tang, John Chu-Tay, Livingston, New Jersey 07039 (US); Naveh, DAvid, Berkeley, CA 94701 (US); Nagabhushan, T.L., Parsippany, New Jersey 07054 (US); Raman, Jay, West Windsor, New Jersey 08691 (US)
(74) Representative: Ritter, Stephen David

(56) References cited:
- WO-A-91/18919
- US-A- 4 723 000

## Description

### BACKGROUND OF THE INVENTION

This invention relates to a method of purifying interleukin-4.

Interleukin-4 (IL-4) is a lymphokine (stimulator of the immune system) that has a broad range of immune-cell stimulatory activities [Banchereau et al., *Lymphokine Res*. Vol. 6, No. 1:U135 (1987); Yokoto et al., *Proc. Natl. Acad. Sci. USA*, 83: 5894-5898 (1986); Lee at al., *Proc. Natl*. *Acad. Sci*. *USA*, 83: 2061-2065 (1986); Coffman et al., *J. Immunol*. 136: 949-954 (1986); Sanderson et al., *Proc. Natl. Acad. Sci. USA*, 83: 437-440 (1986); Grabstein et al., *J. Exp. Med*., 163: 1405-1413 (1985); and Vitetta et al., *J. Exp. Med*. 162: 1726-1731 (1985)]. At various times, IL-4 has also been referred to as B-cell growth factor (BCGF) [Butler et al., *J. Immunol*. 133: 251-255 (1984)(human BCGF); and Farrar et al., *J. Immunol*. 131: 1838-1842 (1983)(mouse BCGF)] and B-cell stimulatory factor 1 (BSF-1) [Ohara et al., *J. Immunol*. 135: 2518-2523 (1985)]. The name interleukin-4 was finally proposed and adopted in 1986 [Sanderson et al., *Proc. Natl*. *Acad. Sci*. *USA*, 83: 437-440 (1986)].

IL-4 was originally thought to be important only for the costimulation of activated B-cells [Roehm, et al., *J. Exp. Med*. 160:679-694 (1984)]. It has also been shown, however, to modulate the activities of T-cells and mast cells [Mosmann, et al., *Proc. Natl. Acad. Sci*, *USA*. 83:5654-5658(1986)]. See also WO 87/0290, where the activity of IL-4 as a T-cell growth factor and B-cell growth factor is described as being useful to enhance natural defenses against various infections.

T-cells and B-cells act in the later stages of an immune response. It would be highly advantageous to have an agent which would increase and activate neutrophils which act in the early stages of infection and are the body's first line of defense against infection.

In the normal process of white blood cell hematopoiesis, cells originate in the bone marrow from a primitive immature cell known as a stem cell and differentiate through progressively more mature stages along different lineage pathways, to arrive at a terminal state of differentiation as a monocyte, granulocyte or lymphocyte.

A property of immature, undifferentiated cells is the ability to multiply rapidly. It is only when a precursor cell matures and differentiates through these multiple stages that it generally loses its capacity to proliferate and assumes the role of a specialized, functional mature cell. In the normal state, cells that reach their final mature form do not proliferate to any great extent, if at all.

In general, cancer is a disorder of cell differentiation. In particular, myeloid leukemias are disorders in which cells of monocytic and granulocytic lineages are blocked at an early stage of maturation and thus have not lost their proliferative capacity. Because these maturation-arrested cells continue to proliferate, they give rise to a population of immature cancer cells, resulting in a diagnosis of leukemia.

There is evidence that various myeloid leukemia cells can be induced to differentiate into normal macrophages and granulocytes, and that upon differentiation these cells lose their capacity to proliferate. This suggests that the induction of terminal differentiation by an agent would be useful as a therapy for myeloid leukemia.

### SUMMARY OF THE INVENTION

The present invention provides an improved process for purifying 1L-4.

According to the invention that is provided a process for purifying a crude solution of active recombinant human interleukin-4, comprising
(a) charging said crude solution of IL-4 buffered at a neutral to slightly alkaline pH and containing about 0.5-1.5M sodium chloride to a metal chelating agarose gel chromatography column to selectively bind the active recombinant human interleukin-4 to the column;
(b) washing said column twice, first with an equilibration buffer containing 1.0M sodium chloride and then with a buffer containing 10% glycerol and 0.15M sodium chloride;
(c) eluting the bound active recombinant human interleukin-4 from the column with an eluting buffer at about pH 4.5 to 5.5;
(d) charging the active human IL-4 solution from (c) in a buffer to chromatography on a cation exchanger and gradient eluting a buffered solution of the active human IL-4 from the column;
(e) concentrating the eluate from (d) on a stirred cell membrane that allows matter of less than 10,000 molecular weight to pass;
(f) subjecting the concentrate from (e) to size exclusion chromatography; and
(g) recovering the purified solution of active recombinant human interleukin-4.

High purity active IL-4 can be obtained from the fermentation broth of IL-4 expressing E. coli in a three-step process comprising :
1.Subjecting crude fermentation broth to affinity chromatography on a metal chelating-agarose gel column such as a chelating-Sepharose® gel available from Pharmacia Fine Chemicals, Piscataway, New Jersey under the names chelating-Sepharose® Fast Flow and chelating-Sepharose® 6B. Chelating Sepharose® Fast Flow consists of iminodiacetic acid groups on spacers coupled to Sepharose 6 Fast Flow by stable ether linkages. Sepharose® 6 Fast Flow is a crosslinked agarose, 6%. A buffer, preferably a phosphate buffer, is used. The phosphate buffer used is one with a high sodium chloride concentration, i.e. about 0.5 to 1.5M, preferably 1.0M, in a neutral to slightly alkaline pH, i.e. about pH 6.7-8, preferably about 7.2. The high salt concentration and near neutral pH of about 7.2 helps maximize binding of the active interleukin-4 and minimize binding of other proteins to the column. The preferred metal chelate is zinc, although other metal chelates such as copper, cobalt or nickel can be used. After the binding is completed, the column is washed twice, first with an equilibration buffer containing 20 mM sodium phosphate, pH 7.2 and 1.0 M sodium chloride. It is then washed with the phosphate buffer containing about 10% glycerol and a low concentration of sodium chloride, i.e. about 150 mM, in a 20 mM sodium phosphate buffer. The second washing removes impurities including very closely related hard to separate impurities. Finally, the active IL-4 is eluted at pH 5.0 with an acetate buffer containing 0.50 M sodium chloride.
2. Subjecting the solution of active IL-4 from step 1 to cation exchange chromatography on a S-Sepharose® Fast Flow column at a near neutral pH of about 6.75 and at 15 mS(conductivity) where most impurities do not bind. The further purified IL-4 in a buffered solution is then eluted from the column; and
3. Subjecting the active IL-4 solution to gel filtration chromatography on a size exclusion column equilibrated with 10 mM sodium citrate, pH 4.5 after concentration up to 20 mg/ml at pH 4.5. Then collecting the purified active IL-4 solution which is 95%-99% pure.

Also, high purity active IL-4 can be obtained from the cell culture medium of IL-4 expressing CHO-cell lines in a process comprising :
1. Subjecting crude cell culture medium containing active IL-4 to cation exchange chromatography on a S-Sepharose® Fast Flow column at a near neutral pH of about 6.7 to 8, preferably 7.2, and at 13-15 mS(conductivity) where most impurities do not bind. S-Sepharose® Fast Flow available from Pharmacia Fine Chemicals, Piscataway, New Jersey is a cross-linked agarose matrix having coupled thereto the ion exchange group -CH₂-SO₃ ⁻ Na⁺. The column is washed with equilibration buffer then the purified IL-4 in a buffered solution is isocratically eluted from the column by a buffer system at pH 7.2, containing 0.26M NaCl and pooled;
2. Subjecting the pooled eluate from step 1 to further cation exchange chromatography on a relatively small S-Sepharose® Fast Flow column which is about 15% the bed volume of the column of step 1. The column is washed with an equilibration buffer, then the active IL-4 molecule is eluted by a buffer system at pH 7.2 containing a sodium chloride gradient 0.12 - 0.50M;
3.Subjecting the solution of active IL-4 from step 2 to affinity chromatography on a metal chelating-agarose gel column after adjusting the solution to pH 7.2 and conductivity to 45-50mS. The chelating-Sepharose® gel is available from Pharmacia Fine Chemicals, Piscataway, New Jersey under the names chelating-Sepharose® Fast Flow and chelating-Sepharose® 6B. Chelating Sepharose® Fast Flow consists of iminodiacetic acid groups on spacers coupled to Sepharose 6 Fast Flow by stable ether linkages. Sepharose® 6 Fast Flow is a crosslinked agarose, 6%. A buffer, preferably a phosphate buffer, is used. The phosphate buffer used is one with a sodium chloride concentration of about 0.5 M at a neutral to slightly alkaline pH, i.e. about pH 6.7-8, preferably about 7.2. The salt concentration and near neutral pH of about 7.2 helps maximize binding of the active interleukin-4 and minimize binding of other proteins to the column. The preferred metal chelate is cobalt although other metal chelates such as zinc, copper or nickel can be used. After the binding is completed, the column is washed with an equilibration buffer containing 20 mM sodium phosphate, pH 7.2 and 0.5 M sodium chloride. Finally, the active IL-4 is isocratically eluted at pH 6.0 with a phosphate buffer containing 0.50 M sodium chloride;
4. Subjecting the active IL-4 solution to gel filtration chromatography on a size exclusion column, preferably Sephacryl® S-200 HR or S-100 HR which are crosslinked copolymers of allyldextran and N,N'-methylene bisacrylamide which are available from Pharmacia, equilibrated with 10 mM sodium citrate, pH 4.5 after concentration up to 20 mg/mL at pH 4.5. Then collecting the purified active IL-4 solution which is 95%-99% pure.

### BRIEF DESCRIPTION OF THE DRAWINGS

The amino acid sequence of a specific (human) IL-4 which may be purified according to the present invention is defined in the Sequence Listing by SEQ ID NO: 1.

### DESCRIPTION OF THE INVENTION

Any suitable IL-4 may be employed in the present invention. Complementary DNAs (cDNAs) for IL-4 have recently been cloned and sequenced by a number of laboratories, e.g., Yokoto et al., *Proc. Natl. Acad. Sci. USA, 83:* 5894-5898 (1986) (human); Lee at al., *Proc. Natl. Acad. Sci. USA,* 83: 2061-2065 (1986)(mouse); and Noma et al., *Nature 319:* 640-646 (1986)(mouse). Le et al., J. Biol. Chem. 263: 10817 (1988) have described the production of recombinant human IL-4 in CHO cells. IL-4 is also an article of commerce, available, e.g., from Genzyme Corporation, Boston, Massachusetts (human and mouse). Moreover, non-recombinant IL-4 has been purified from various culture supernatants, e.g., Sanderson et al., *Proc. Natl. Acad. Sci. USA, 83:* 437-440 (1986)(mouse); Grabstein et al., *J. Exp. Med.,* 163: 1405-1413 (1985)(mouse); Ohara et al., *J. Immunol.,* 135: 2518-2523 (1985)(mouse BSF-1); Butler et al., *J.* Immunol., *133:* 251-255 (1984)(human BCGF); and Farrar et al., *J. Immunol.,* 131: 1838-1842 (1983) mouse BCGF).

Preferably, the IL-4 used in the present invention will be a human IL-4, and most preferably it will be the human version with the sequence described in Yokoto et al., *Proc. Natl. Acad. Sci.* USA, 83: 5894-5898 (1986) and PCT patent application no. 87/02990 published May 21, 1987.

### Construction of the Human IL-4 Expression Plasmid odhfr-SRalpha263

The construction of plasmids pSRα-CAT196, pcD137, pcD-SRα205, pcDhlL-4 clone 125 and pcD-SRα224 have been described (Takebe et al., *Molecular and Cellular Biology,* 8: 466-477 (1988); and Yokoto et al., *Proc. Natl. Acad. Sci. USA,*83: 5894-5898 (1986)). As shown in Figure 1, plasmid pcD-SRα-205 was constructed by the trimolecular ligation of the 373bp Ncol-Xhol SRα promoter fragment from pSRα-CAT196, the 434bp Xhol-Sstl splice junction (SJ) and 5' murine IL-4 (mIL-4) fragment from pcD137 and the 3221bp Sstl-Ncol fragment, also from pcD137, containing the 3' murine IL-4 cDNA, SV40 polyadenylation region and the pBR322 derived plasmid backbone containing the origin of replication and ampicillin resistance gene. The G-C tail was deleted from pcD-hIL-4 clone 46 (Yokota et al., *Proc. Natl. Acad. Sci. USA*, 83: 5894-5898)) as follows. The Okayama-Berg plasmid pL1 (Okayama and Berg, *Molecular and Cellular Biology*, 3: 280-289 (1983)) was restricted with PstI and the four nucleotide overhang removed by the 3'-5' exonuclease activity of T4 polymerase. BgIII linkers were ligated to the flush DNA ends followed by restriction with BgIII and HindIII. The HindIII BgIII fragment containing the SV40 sequence of pL1 was isolated and inserted into BgIII/HindIII restricted pcD-MCGF (Yokoto et al., *Proc Natl. Acad. Sci, USA*, 81: 1070-1074 (1984)) to yield intermediate plasmid 101. The purified 3IIbp Pst fragment from plasmid pcD-hIL-4 clone 46 was restricted with Sau3A-I which releases a 163bp fragment with overhangs compatible with BgIII. The 162bp fragment was ligated to BgIII restricted p101 to yield intermediate 112. The HindIII/NheI fragment of p112 containing SV40 and human IL-4 cDNA sequences was ligated to HindIII/NheI restricted clone 46 DNA to produce pcD-hIL-4 clone 125 containing an SV40 early promoter, SV40 splice junction and complete human IL-4 cDNA with the G-C tail deleted. Plasmid pcD-Srα224 was constructed by replacing the small XhoI fragment of pcD-SRα205 (containing the SJ and mIL4 cDNA) with the small XhoI fragment of pcDhIL-4 clone 125 containing the SJ and HIL-4 cDNA with G-C tail deleted as described above.

A SalI site was introduced into pMTVdhfr (Lee et al., *Nature*, 294: 228-232 (1981)) by EcoRI/BamHI restriction, Klenow polymerase fill in of the overhang and ligation to an octanucleotide SalI linker as shown in Figure 1. Plasmid pMTVdhfr259, then, lacks restriction sites for EcoRI and BamHI and the region between the two is replaced with a SalI linker. The SalI fragment of pcD-SRα224 containing the Srα promoter, SV40 SJ, human IL 4 cDNA and SV40 polyadenylation signals was inserted into the unique SalI site of pMTVdhfr259. The final human IL-4 expression plasmid, pdhfr-SRalpha263 contains the following elements, counterclockwise from the SalI site:
1. Ampicillin resistance gene and origin of replication from pBR322.
2. MMTV LTR driven dhfr expression unit from pMTVdhfr.
3. SRalpha promoter.
4. SV40 derived splice junction.
5. Human IL-4 cDNA.
6. SV40 derived polyadenylation signal.

The human IL-4 cDNA sequence present in the vector is the same as in pcD-HIL-4 clone 46 given in Yokoto et al., *Proc. Natl. Acad. Sci. USA*, 83: 5894-5898(1986).

### DHFR Gene Amplification and Selection of IL-4 SI Line

Chinese hamster ovary cell mutants deficient in dihydrofolate reductase activity (CHO-dhfr⁻) are widely used for overproduction of various recombinant proteins. (Kaufman et al., *Molecular and Cellular Biology*, 5: 1750-1759 (1985)). CHO-dhfr⁻ mutant cells have an auxotrophic requirement for hypoxanthine, thymidine and glycine. Expression vectors incorporating a dhfr marker may be used to complement this mutation; selection is achieved by growing cells in the absence of the required media cofactors described above. Gene amplification (increase in copy number up to 1000X) may be accomplished by growing cells in increasing concentration of the folate analog methotrexate (MTX). The amplification of the integrated recombinant dhfr locus in the genome results in a concomitant increase in copy number of the expression unit for the gene of interest (Ringhold et al., *J. Mol. Applied Genetics*, 1: 165-175 (1981); and Kaufman et al., *EMBO J*., 6: 187-193 (1987)).

The plasmid DNA having the coding sequence for dhfr and human IL-4 (pdhfr-SRα263) was constructed as described above. Transfection of pdhfr-SRα263 into DXB-II CHO-dhgr⁻ cell line was carried out by the calcium phosphate precipitation method. (Graham and Van der Eb, *Virology*, 52: 546 (1978)) Transformants were selected in a selection medium (DMEM, Dulbecco's Modified Eagle's Medium) that lacks hypoxanthine and thymidine. A clone designated 3B12 was chosen for the first cycle of amplification. The 3B12 clone was cultured in α-MEM medium (Eagle's minimum essential medium) containing 40 nM MTX until resistant clones were selected. A clone designated 3B12-A26 was used for further amplification with 1µm MTX. After the second cycle of drug selection, a clone designated 3B12-A26-19 was chosen for further development. This clone was adapted to growth in a suspension mode with 10% NU Serum™ V and a subclone designated IL-4 SI was selected for the large scale propagation.

### Culture Preparation

In order to prepare a Master Cell Bank (MCB), two original 100ml spinner flasks containing the IL-4 SI cells were used. The cells were carried through two additional growth medium exchanges and grown in 100 ml spinner flasks (growth medium is basal medium plus 0 to 10% serum, e.g., NU Serum™ V). Cells from each flask were collected, washed, resuspended in 10 ml of freezing medium, pooled and aseptically dispensed in about 2.0 ml sterile cell storage vials (freezing medium is basal medium plus 20% serum, e.g., NU Serum™ V plus 10% dimethylsulfoxide). The vials were slowly frozen at -70°C and stored in liquid nitrogen.

The cells from three frozen vials were thawed and propagated by suspension in growth medium for 4 to 6 generations in spinner flasks of increasing volume from 100 ml up to 3 liters. Cells were collected by centrifugation, washed, and resuspended in freezing medium. The cell suspension was aseptically dispensed in about 2.0 ml sterile cell storage vials. The vials were slowly frozen at -70°C and stored in liquid nitrogen to constitute the Master Cell Bank (MCB).

A Master Working Cell Bank (MWCB) was prepared from the MCB by thawing 1 to 3 vials of the MCB and propagating the cells in T-flasks and in suspension for 4 to 6 generations in increasing volumes up to 3 liters. Cells were collected, washed, resuspended in freezing medium and aliquoted and frozen as described for the MCB. The MWCB was stored in liquid nitrogen as well.

IL-4 production was carried out in bioreactors of 50 to 200 liters in volume, To start production, one frozen vial from the MWCB was thawed and inoculated into a T-75 flask. From incubation until cell concentration reaches 100% confluency, cells were trypsinized and inoculated into two T-75 flasks (optionally, a T-160 flask can be used). These flasks were again incubated until 100% confluency and the trypsinized cells were used to inoculate a 100 ml spinner flask.

The 100 ml spinner flask was incubated until adequate cell growth was obtained and was used as inoculum for a 250 ml spinner flask. A similar step was repeated in a 1 liter and a 3 liter flask and a 10 to 20 liter bioreactor. Cells from the 10 to 20 liter reactor were used as inoculum for a 50 to 100 liter reactor. This reactor is initially grown batchwise and upon achieving adequate cell concentration, a continuous media perfusion was initiated.

The media used for growth and continuous perfusion was modified Iscove's medium, which may be supplemented with up to 10% (e.g., NU Serum™ V). No methotrexate was used throughout the production process.

The fermentation stages were carried out under sterile conditions and in closed systems. The key fermentation parameters such as temperature, pH, agitation and aeration were monitored and controlled as appropriate throughout the growth and continuous perfusion stages. Aseptic samples were taken periodically to measure pH, cell density and to check for sterility (absence of bacteria and fungi).

Upon collection of an adequate volume of conditioned media (perfusate), the broth was filtered to remove any cells that may be present, and concentrated via ultrafiltration. The concentrate, which contains crude CHO IL-4, was forwarded to the final purification stages.

Purification of IL-4 from the crude CHO IL-4 concentrate was carried out by performing a cation exchange chromatography on a sulphonate column (e.g., S-Sepharose). This step was typically repeated. Selected pooled fractions from the sulphonate column were then forwarded to a chelate chromatography step (e.g., cobalt-chelate Sepharose). The selected pooled chelate fractions were then diafiltered and concentrated via membrane filtration. The concentration was chromatographed in a gel filtration column (e.g., HR S-200). The pooled fractions which constitute the purified bulk IL-4 were then filtered and stored at -20°C or lower.

The process for purifying such a crude CHO-IL concentrate of active recombinant human IL-4 thus comprises:
(a) subjecting a buffered crude solution of active IL-4 from aCHO-cell culture medium to cation exchange chromatography at a near neutral to a slightly alkaline pH to selectively bind the IL-4, and isocratically eluting the IL-4;
(b) subjecting the eluate from step (a) to further cation exchange chromatography on a relatively small column(15% of the bed volume of step(a)) at a near neutral to slightly alkaline pH and gradient eluting the IL-4;
(c) subjecting the eluate from step (b) to affinity chromatography on a cheating agarose gel column system at a near neutral to slightly alkaline pH, then eluting the IL-4 with an acidic buffer;
(d) concentrating the eluate from step (c) with an ultrafiltration membrane( 10,000 molecular weight cut-off); and
(e) subjecting the concentrated solution of active IL-4 from step (d) to gel filtration chromatography on a size exclusion column at an acid pH and collecting the purified IL-4 solution.

The cation exchange chromatography is carried out in two steps after the CHO-cell culture medium is filtered to remove extraneous large cell debris, then concentrated to up to 100mg/mL protein on a diafiltration membrane and the pH is adjusted to pH 7.1-7.3, preferably 7.2. The membrane is preferably a stirred cell fitted with a membrane which holds all material greater than 10,000MW, e.g. a YM-10 membrane, Amicon Co., U.S.A.,or Pellicon filter PTGC Cassettes, Millipore Corp., Bradford, Mass. In the first step, crude culture medium is loaded on a cation exchange chromatography column such as S-Sepharose® Fast Flow ( up to 100 mg protein per ml resin) previously equilibrated with a phosphate buffer of near neutral to slightly alkaline pH, i.e. pH 6.7-8, preferably 7.2, containing 0.12M sodium chloride. This results in the active IL-4 remaining on the column and most undesired proteins and other impurities being washed through. The active IL-4 is then isocratically eluted from the column with a sodium phosphate buffer, preferably one of pH 7.1-7.3, more particularly, pH7.2, with 20mM sodium phosphate and about 0.26M sodium chloride. The fractions containing active IL-4 as determined by the SDS-PAGE and protein assays are pooled and the pool is adjusted to pH 7.2 and 14mS.

In the second step, the adjusted pool from the first step is loaded on a relatively small cation exchange chromatography column, about 15% bed volume of the cation exchange column used in step 1, equilibrated with a phosphate buffer, impurities are washed through, and the active IL-4 remains on the column. The active IL-4 is eluted with a sodium chloride gradient of 1.75mS per bed volume. The eluting buffers consist of a low salt buffer, i.e. 20mM sodium phosphate, pH 7.2, 0.12M sodium chloride and a high salt buffer, i.e. 20mM sodium phosphate, pH 7.2, 0.50M sodium chloride. The gradient fractions containing the active IL-4 as determined by SDS-PAGE and protein assays are pooled and adjusted to pH7.2 and 45-50 mS.

The pool of active IL-4 fractions from the cation exchange chromatography which is about 60 - 70% pure is then subjected to affinity chromatography on a metal cheating agarose gel column prepared by metal treated chelating-Sepharose® gel, i.e. chelating-Sepharose®Fast Flow or chelating-Sepharose®6B. The chelating columns comprise two portions in a single column. The top part of the column contains a metal treated-cheating agarose gel, preferably a cobalt treated-chelating-Sepharose®Fast Flow gel, and the bottom part of the column is an untreated chelating-Sepharose®Fast Flow gel. The volume ratio of the two layers is about 2.3-3.0 volumes of cobalt treated-chelating Sepharose® to one volume of untreated chelating Sepharose®. When the pool of purified IL-4 from the cation exchange treatment is loaded unto the top of the column, the solution flowing through traverses to the bottom portion of thecolumn where the flow-through containing any remaining impurities exits.

By using a buffer at near neutral, preferably pH 7.2, and preferably about 0.5 M sodium chloride, active IL-4 molecules are selectively bound by affinity chromatography to a metal chelating-agarose gel column, preferably cheating Sepharose® Fast Flow or Sepharose® 6B, to the substantial exclusion of contaminating proteins present in the solution. The active IL-4 remains on the columns and is isocratically eluted with a buffer at a slightly acid pH, preferably at pH 6.0 containing 0.5M NaCl.

The purified solution of active IL-4 from the affinity chromatography column is concentrared on an ultrafiltration membrane(10,000 MW cut-off), preferably on a stirred cell fitted with a membrane which holds all material with greater than 10,000 molecular weight, which range includes IL-4. A preferred membrane is YM-10 manufactured by Amicon Co., USA. The concentration obtained is up to 20mg/mL. Two diafiltration buffers are used, first a 20mM Na-phosphate buffer at pH 6.0 with 0.5 M sodium chloride, and a second buffer which is preferably 10mM sodium citrate at pH 4.5.

The concentrated eluates of active IL-4 are charged to a size exclusion gel filtration column which fractionates the proteins in the solution according to molecular weight. A typical column which is suitable is a Sephacryl®S-200 HR or S-100 HR (Pharmacia) gel filtration column. The Sephacryl®S-200 HR(high resolution) and S-100 HR are crosslinked copolymers of allyldextran and N,N'-methylene bisacrylamide. Their fractionation range in Daltons is 5,000 -250,000 and 1,000 - 100,000, respectively. Other suitable materials are the Sephadexes® (Pharmacia) which are crosslinked dextran gels. Preferably, the solution of active IL-4 is charged to an S-200 HR column previously equilibrated with a 10 mM citrate buffer at pH 4.5

A more preferred embodiment comprises :
(a) subjecting a crude buffered solution of active recombinant human IL-4 from a CHO-cell cell culture medium to cation exchange chromatography on a cross-linked agarose column (preferably S-Sepharose®Fast Flow) in a 20mM phosphate buffer, pH 6.7 to 8, preferably, pH 7.2, with 0.12M sodium chloride at 13-15 mS, preferably 14mS, then isocratically eluting the active IL-4 from the column with a 20mM phosphate buffer at pH7.1 to 7.3, preferably, pH 7.2 with 0.26M sodium chloride and collecting the active IL-4 fractions;
(b) subjecting the IL-4 solution from step (a) to additional cation exchange chromatography on the same type of cross-linked agarose column used in step (a) but having a bed volume of about 15% of the column used in step (a), in a buffer at pH7.2 - 7.5, preferably 7.2, containing 0.12M sodium chloride, then gradient eluting with a 20mM phosphate buffer at pH 7.2 containing 0.12 M to 0.50M sodium chloride and pooling the fractions containing active IL-4;
(c) subjecting the pooled fractions of active IL-4 from step(b) at pH 7.2 to affinity chromatography on a metal cheating agarose gel column, preferably consisting of a top portion of a cobalt-chelating Sepharose®Fast Flow or 6B gel and a bottom portion of untreated chelating Sepharose®Fast Flow gel, with a 20mM phosphate buffer at pH 7.2 containing 0.5M sodium chloride, the volume ratio of the two portions is about 2.3-3.0 volume of cobalt-treated chelating Sepharose® to one volume of untreated cheating Sepharose®, washing with an equilibration buffer, then eluting the active IL-4 with a phosphate buffer at pH 6.0 containing 0.5M sodium chloride and collecting the IL-4 fractions; and
(d) concentrating and diafiltering the IL-4 fractions(pooled) from step (c) to up to 20mg/mL at pH4.5 on an ultrafiltration membrane which holds all material with greater than 10,000 molecular weight, preferably a stirred cell fitted with a membrane such as YM-10, then subjecting the active IL-4 concentrate to size exclusion (gel filtration) chromatography on a column which fractionates the proteins in the solution according to molecular weight on a cross-linked copolymer of allyldextran and N,N'-methylene bisacrylamide, preferably Sephacryl®S-200 HR in a 10mM sodium citrate buffer at pH 4.5 and collecting the active IL-4 fractions.

In step (a) and step (b) the pH of the fractions are adjusted to pH 7.2 and a conductivity of 13-15mS with 4M NaCl. The bed volume ratio of the two cation exchange columns is about 6.3 volume of S-Sepharose® column in step (a) and one volume of the cation exchange column in step (b). The cation exchange gel material in a chromatography column is equilibrated with a 20 mM phosphate buffer at pH 7.2 having 0.12 M sodium chloride. A preferred cation exchanger is crosslinked agarose substituted with -CH₂-SO₃ ⁻ Na⁺ groups, such as S-Sepharose® Fast Flow available from Pharmacia. The active IL-4 is isocratically eluted in step (a) with a 20 mM phosphate buffer at pH 7.2 with 0.26M NaCl. The fractions with highest concentrations of active IL-4 based on the SDS-PAGE and protein assays are pooled. The pooled fractions solution is adjusted to pH 7.2 and the conductivity is adjusted 13-15mS per bed volume with 20mM sodium phosphate buffer pH 7.2 The column is then loaded with the IL-4 solution and gradient eluted using a gradient of 1.75mS with 20mM sodium phosphate buffers of pH 7.2 containing 0.12-0.50M NaCl. The collected IL-4 containing fractions as determined by the SDS-PAGE and protein assays are pooled. The conditions for cation exchange chromatography are selected to insure that the active IL-4 fraction will attach to the cation exchanger matrix. The near neutral pH 7.2 which is relatively high for a cation exchange chromatography and the 13-15mS conductivity which is relatively high for cation exchange chromatography results in mild binding conditions where most impurities do not bind to the column, elution is relatively easy and high purity of the active IL-4 solution is obtained, i.e. about 60%-70%.

The preferred metal chelating-agarose utilized in step (c) is chelating Sepharose® Fast Flow, although chelating Sepharose®6B is also satisfactory. The Sepharoses are products of Pharmacia Fine Chemicals, Piscataway, New Jersey. A preferred method of preparing the preferred cobalt cheating Sepharose® column for use in this invention is by suspending the Sepharose® gel in 0.02 M cobalt acetate solution and washing with deionized water, then an equilibration buffer, i.e. 20mM sodium phosphate, pH 7.2, 0.5M NaCl solution through the column. Instead of cobalt acetate, other cobalt salts may be used, e.g. cobalt chloride or cobalt sulfate.

The chromatography column comprises one column containing two layers , the first or top layer contains a metal chelating-Sepharose® gel and the second or bottom layer contains chelating Sepharose® gel which has not been treated with a metal salt. The volume ratio in the dual columns is about 2.3 to 3.0 volumes of metal treated cheating Sepharose® to one volume of untreated cheating Sepharose®. The preferred metal is cobalt.

The preferred buffer used to equilibrate the columns is a 0.02 M phosphate buffer at pH 7.2-7.5 containing 0.5M sodium chloride.

In step (c) the cobalt chelating-Sepharose® and the untreated cheating Sepharose® gels are equilibrated with a phosphate buffer at pH 7.2 containing 0.5M sodium chloride, then the adsorbed active IL-4 is isocratically eluted from the cobalt chelating-Sepharose® through the untreated cheating -Sepharose® with a 0.02 M phosphate buffer at pH 6.0 with 0.5 M sodium chloride or alternatively with a neutral pH buffer containing a 0.5M NaCl and of (i) a chelating agent such as 50mM EDTA(ethylenediaminetetraacetic acid), or (ii) an analog of histidine such as 50mM imidazole, or (iii) an amino acid such as 50mM histidine. Preferred is the phosphate buffer at pH 6.0 with 0.5M NaCl. The eluate containing active IL-4 is collected. The fractions with the highest concentrations of active IL-4 based on the conventional SDS-PAGE and protein assays are pooled.

In step (d) the pooled eluted fractions from step (c) are concentrated and diafiltered then subjected to gel filtration chromatography. The eluted fractions are concentrated on a stirred cell fitted with a membrane which holds all material with greater than 10,000 molecular weight and diafiltered first against a 0.02 M sodium phosphate buffer, pH 6.0 containing 0.05M EDTA (ethylene diamine tetraacetic acid) and 0.5M NaCl, then 0.01M sodium citrate, at pH 4.5. Since, at this stage of the process the active IL-4 solution is about 90-95% pure, that is in the concentrated solution retained on top of the membrane. A preferred membrane is YM-10 manufactured by Amicon Co., U.S.A. The concentration obtained is up to about 20mg/mL of active IL-4. The concentrated eluates of active IL-4 are charged to a size exclusion (gel filtration) column which fractionates proteins in the solution according to molecular weight. A typical column which is suitable is a Sephacryl®S-200 HR or S-100 HR(Pharmacia) gel filtration column. The Sephacryl®S-200HR(high resolution) and S-100 HR are crosslinked copolymers of allyldextran and N, N'-methylene bisacrylamide. Their fractionation ranges are 5,000 - 250,000 and 1,000 - 100,000, respectively. Other suitable materials are the Sephadexes® (Pharmacia) which are crosslinked dextran gels. Preferably the solution of active IL-4 is charged to an S-200 HR column previously equilibrated with a 10 mM sodium citrate buffer at pH 4.5. Under the conditions of step (d) the stable IL-4 concentrate can reach up to 20 mg/ml. This increases the capacity and performance of the gel filtration chromatography. The fractions of eluate containing the highest concentrations of active IL-4 as determined by the SDS-PAGE and protein assay are collected and pooled to result in a 95-99% pure solution of active IL-4.

The above references are hereby incorporated by reference for their relevant teachings of materials and methods used in the construction of the CHO expression system for hIL-4.

### CONSTRUCTION AND CHARACTERIZATION OF THE E. COLI-DERIVED HUMAN IL-4

### A. Construction of the human IL-4 expression plasmid. pRGT857-11

The construction of the human IL-4 expression plasmids, pAH3, pKGT269-2 and pUC 19, have been described previously. Lundell et al., J. Ind. Microbiology, 1989 and Yanisch-Perron et al., Gene, 33:103-119, 1985. In order to construct pRGT857-11, pAH3 was digested with the restriction endonuclease, Aval. The 5' overhang created by this enzyme was filled in with the Klenow fragment of E.coli Pol I, and the DNA was digested with Pvul. The 5.8 Kilobase (KB) fragment carrying the IL-4 and laci regions was ligated to the 1.4 KB Pvull-Pvul fragment of pUC 19, carrying the pUC origin of replication. Following transformation of E.coli 294, one of the ;ampicillin resistant IL-4 expression plasmids carrying the pUC origin of replication was pRGT839-2, as shown in Figure 2.

pRGT839-2 and pKGT269-2 were then both digested with Aatll and Pvul. The 6.7 KB fraagment of pRGT839-2 carrying the IL-4 and laci regions was ligated to the small 1 KB fragment from pKGT269-2,, encoding chloramphenicol resistance. The ligation reaction was used to transform E. coli 294. One of the resulting transformants was pRGT857-11. As shown in Figure 2, this IL-4 expression plasmid carries chloramphenicol resistance as well as the pUC origin of replication. This plasmid was subsequently used to transform E.coli RL7321.

### B. Host Bacterium

The host organism carrying the plasmid, pRGT857-11 which is used for the production of human IL-4 is E.coli K-12. The strain is E. coli RL732l, a derivative of E.coli MM294 which has been previously described by Bolivar et al., Methods in Enzymology, Vol. 68, 245-267, (Ray Wu, ed.), Academic Press, 1979. This strain conforms with the guidelines established for an EK1 host. E.coli RL7321 was isolated from E. coli MM294 as follows:

A streptomycin resistant form of E.coli MM294, designated E. coli 294S, was first isolated by transducing the former strain with bacteriophage P1 cml, clr100 [Miller, J. H., 1972. Experiments in Molecular Genetics. Cold Spring Harbor Laboratory. Cold Spring Harbor, N.Y.] which had been grown on E. coli PAM163 [Johnson, B.F. 1977. Fine structure mapping and properties of mutations suppressing the ion mutation in Escherichia coli K-12 and B strains. Genet. Res., 30:273-86].

E. coli 294S was mutagenized with ultraviolet light to isolate a strain defective in outer membrane structure. The cells were irradiated for 40 seconds with a UV lamp. This treatment caused 99.9% cell death, determined by plating on rich medium. The mutagenized cell suspension was diluted and incubated at 37°C for 3 hours in the dark with shaking.

At his time, T7 wild type bacteriophage were added to 10⁸ plaque forming units per ml. The bacteriophage T7 is used as a selection to enrich for bacteria having mutations in their outer membranes. Since the T7 receptor is located on lipopolysaccharide, an outer membrane protein, some outer membrane mutations will result in resistance to T7 infection. Cells having wild-type outer membranes will be sensitive to infection and thus be killed off. The leaky phenotype, discussed below, is due to an increased permeability of the outer membrane. The use of bacteriophage T7 resistance as an indication of outer membrane damage has been shown previously [Branes et al., J. of Bacteriology, 154:1462-1466, 1983].

Following T7 infection, the flask was shaken at 37°C until cell lysis was observed (approximately 30 minutes). The T7 resistant cells were collected by centrifugation and resuspended in 1 ml fresh broth. These cells were spread onto TYE (Tryptone:yeast extract: sodium chloride@20:10:5) agar plates and incubated at 37°C overnight. After 24 hours, each plate contained from 30-50 colonies. These colonies were for outer membrane damage. One method was to observe any increase in RNase I leakage to the medium. Single colonies of T7 bacteriophage resistant clones were streaked across fresh TYE agar plates that had been overlaid with 4 ml of TYE agar containing 1% yeast RNA (Sigma Corp.) at pH 7. After overnight incubation at 37°C, tee plates were flooded with 1N HC1. Halo size was used to determine strains leaking RNase activity into medium [Weigand, R.A. and Rothfield, J. of Bacteriology, 125:340-345, 1976]. A second indicator of outer membrane damage in E. coli is failure to grow on MacConkey agar [Hancock, R.E.W., Ann. Rev. Microbiol., 38:237-64, 1984]. One of the thirty T7-resistant colonies shown to be particularly sensitive to MacConkey agar and able to release substantial amounts of periplasmic RNase I was designated RL7.

E. coli RL7 was transformed with the huIL4 expression vector pAH3 (Fig. 1). This vector directs the lymphokine across the inner cell membrane into the periplasm. Spent media from transformants was screened for leakiness by western blot analysis using the huIL-4 polyclonal antibody described below. RL7/pAH3 was mutagenized as before with UV light. After growth in the dark for at least 2 hours, the irradiated cells were plated on TYE agar plates supplemented with ampicillin (100 µg/ml) and incubated overnight at 30°C. The colonies werre screened by "double disc assay" for increased release of huIL4 as indicated by a more intense color development under the mutant colonies. The double disc assay is performed as follows:

The mutagenized cells were diluted and spread (0.1 ml per plate) onto TYE agar plates (142 mm diameter) containing 100 µg/ml ampicillin. After incubation at 30°C overnight, the plates contained from 500-2000 colonies of approximately 1 mm diameter. The plates were then covered with a 137 mm nitrocellulose disc (Schleicher and Schuell) with 0.45µ pore size. The disc was gently applied from one edge to allow gradual and even wetting. The disc was immediately peeled back in one motion so that the colonies were lifted from the agar plate onto the nitrocellullose disc. This disc was nitrocellulose disc (or discs) previously placed onto the surface of a sterile agar plate. The plates were then incubated overnight at 30°C. After incubation, the bottom disc (or discs) was separated from the colony bearing disc. The filters wre incubated in 10 mM Tris pH 8, 150 mM NaCl and 0.05% (v/v) Tween-20 (Bio Rad, Enzyme lmmuno Assay Purity) (TBST) containing 1% BSA (bovine serum albumin) at room temperature for 60 minutes. Filters were then incubated with either rabbit polyclonal antiserum (1:1500 dilution in TBST/BSA; used for dertermination of total huIL-4) or monoclonal antiserum (11B4) (1:10 dilution of hybridoma culture supernatant in TBST/BSA; used for determination of protein in a native conformation) at room temperature for 30 minutes. The filters were washed three times in TBST, then incubated with the appropriate alkaline phosphatase-linked secondary antibody for 30 minutes. The filters were washed three times and stained with an alkaline phosphatase substrate (ProtoBlot System by Progmega Biotec). Slots were then aligned with stock plates and colonies showing increased huIL4 specific staining were selected.

The suspect colonies were cured of the plasmid by continuous transfer in non-selective media followed by streaking on non-selective TYE plates. Colonies that scored negative for growth on ampicillin plates were checked for absence of plasmid and then retransformed with the human IL-4 expression plasmid, pRGT857-11. These clones were screened for increased release of huIL-4 by dot immuno-blotting whole broths obtained from 10 ml tube fermentations. Detection was by a monoclonal antibody to human IL-4 (11B4) followed by alkaline phosphatase conjugated, goat anti-rat IgG. One strain selected as a high producing strain was designated RL731.

E. coli RL731/pRGT857-11 was mutated with UV light as before. After the requisite growth in the dark, the cells were plated on TYE agar supplemented with antibiotic and 1 mM IPTG (isopropyl β-D thiogalactoside). RL731/pRGT857-11 does not grow in the presence of the inducing substance, IPTG. Cells were plated at a density of 10⁴ colony forming units per ml. About 5-10 colonies per plate developed upon overnight incubation. Over 75 colonies were purified by streaking and checked for huIL-4 production. Production level was determined by western blot analysis. Clones that showed the heaviest bands were cured of their plasmid as before, retransformed with pRGT857-11 and checked for retainment of high production of huIL-4. The strain picked for most satisfactory characteristics, including production and leakage of biologically active huIL-4 and continued cell growth after the induction of huIL-4 expression, was RL732I.

During the fermentation of human Interleukin-4 (rhIL-4), the product is directly secreted into the fermentation broth. Initial downstream steps are performed to separate the broth fron the cells and then concentrate the rhIL-4 in the broth. There are two distinct processes to accomplish this task: a membrane process and a sodium trichloroacetic acid (TCA) process. In the sodium TCA process, cells are inactivated by addition of TCA salt. After removing the cells, the pH of the broth is lowered to precipitate the rhIL-4, and the broth is centrifuged to recover rhIL-4 in the form of a pellet or sludge. The membrane process uses both microfiltration and ultrafiltration to recover rhIL-4 in the form of a liquid concentrate. This process may employ several washes with buffer to enhance downstream recovery.

Purification of rhIL-4 from a crude concentrate of the fermentation broth is carried out by performing an immobilized metal affinity chromatography on a metal chelate column (e.g. with Zn-Sepharose). Selected fractions are pooled and forwarded to a cation exchange chromatography step on a sulphonate column (e.g. with S-Sepharose).

The selected fractions are pooled, concentrated and diafiltered with an ultrafiltration apparatus containing a membrane of appropriate nominal molecular weight so that the product remains in the concentrate (e.g. with Millipore PTGC ultrafiltration membranes). The diafiltered concentrate is further purified by chromatography on a gel filtration column (e.g. with S-200 HR). The pooled fractions which constitute the purified bulk rhIL-4 are then filtered and stored at -20°C or below.

The process for purifying a crude solution of active recombinant human IL-4 from E. coli thus comprises.
(a) subjecting a buffered crude solution of said IL-4 at a neutral to a slightly alkaline pH and containing about 0.5 to 1.5 molar sodium chloride to affinity chromatography on a metal chelating-agarose gel to selectively bind the IL-4;
(b) washing the column first with a 20mM sodium phosphate equilibration buffer at pH 7.2 containing 1.0 M sodium chloride, then with the buffer containing 10% glycerol by volume and about 150 mM of sodium chloride;
(c) eluting the bound IL-4 with an eluting buffer at an acid pH or a neutral pH buffer containing a chelating agent, an analog of histidine or an amino acid;
(d) treating the active IL-4 eluate from step (c) with a cation exchange chromatography column such as S-Sepharose® (available from Pharmacia Fine Chemicals) at a near neutral to slightly acid pH and at a conductivity of 15 mS, S-Sepharose® is a cross-linked agarose matrix having coupled thereto the ion exchange group -CH₂-SO₃ ⁻ Na⁺;
(e) concentrating the eluate from step (d) with an ultrafiftration membrane( 10,000 molecular weight cutoff);
(f) treating the concentrated retentate by gel filtration chromatography on a size exclusion column; and
(g) collecting the purified active IL-4.

The most preferred embodiment comprises the following steps:
(a) charging a crude solution of active recombinant human IL-4 to an affinity chromatography column of a metal chelating-agarose gel, preferably chelating Sepharose® Fast Flow, in a neutral to slightly alkaline pH phosphate buffer containing 1.0M sodium chloride;
(b) washing the column twice, first with a phosphate buffer at pH7.2 - 7.5 containing 1.0M sodium chloride, then with the phosphate buffer containing 10% by volume of glycerol and 150mM sodium chloride;
(c) isocratically eluting the active IL-4 from the column with an eluting acetate buffer at pH 5.0 containing 0.5M sodium chloride;
(d)subjecting the active IL-4 eluate in a phosphate buffer at pH 6.75, conductivity 15mS, from step (c) to cation exchange chromatography on a column such as S-Sepharose® equilibrated with 20mM sodium phosphate buffer, pH 6.75 and 0.12M sodium chloride;and gradient eluting with a phosphate buffer at pH 6,75 having 0.12-0.6M NaCl;
(e) concentrating the eluate from step (d) with an ultrafiltration membrane;
(f) treating the concentrated eluate by gel filtration chromatography on a size exclusion column eqilibrated with 10mM sodium citrate buffer at pH 4.5; and
(g) collecting the purified active IL-4 solution.

The preferred E. coli strains used to prepare the active IL-4 purified according to this invention are RL 2117/pRGT857-11 and RL732I/pRGT857-11.

The preferred metal chelating-agarose utilized in step (a) is chelating Sepharose® Fast Flow although chelating Sepharose®6B is also satisfactory.The Sepharoses are products of Pharmacia Fine Chemicals, Piscataway, New Jersey. A preferred method of preparing the preferred zinc cheating Sepharose® column for use in this invention is by pouring the Sepharose® gel into a chromatography column, washing with deionized water, then pumping a salt, preferably zinc acetate, solution and deionized water through the column, then pumping an equilibration buffer, i.e. 20mM sodium phosphate, pH 7.2, 1.0M NaCl solution through the column. Instead of zinc acetate, other zinc salts may be used, e.g. zinc chloride or zinc sulfate.

The chromatography column is two columns connected in series. The first or top column contains the zinc chelating-Sepharose® gel and the second or bottom column contains chelating Sepharose® gel which has not been treated with a zinc salt or other metal salts. The volume ratio in the dual columns is about three volumes of zinc treated chelating Sepharose® to one volume of untreated chelating Sepharose®.

The preferred buffer used to equilibrate the columns is a phosphate buffer at pH 7.2-7.5 containing 1.0M sodium chloride.

In step (b) a special two-part wash with first as an equilibration buffer the phosphate buffer at pH7.2 containing 1.0M sodium chloride then with the sodium phosphate buffer at pH 7.2-7.5 containing10% glycerol and a low concentration of sodium chloride (150mM) are charged to the column. The washes remove impurities, including one which is very closely related and difficult to separate. The active IL-4 remains on the column.

The preferred buffer to maintain the pH of the solution of active IL-4 is a phosphate buffer at pH 7.2 containing 1.0M sodium chloride. When the buffered crude active IL-4 solution is run through the columns, the IL-4 is selectively adsorbed on to the gels.

In step (c) the zinc chelating-Sepharose® and the untreated chelating Sepharose® gels are equilibrated with a phosphate buffer at pH 6.75 containing 1.0 M sodium chloride then the adsorbed active IL-4 is isocratically eluted from the zinc chelating-Sepharose® through the untreated chelating -Sepharose® with an acetate buffer at pH 5.0 with 0.5 M sodium chloride or alternatively with a neutral pH buffer containing a chelating agent such as 50mM EDTA(ethylenediaminetetraacetic acid) or an analog of histidine such as 50mM imidazole, or an amino acid such as 50mM histidine. Preferred is the acetate buffer. The eluate containing active IL-4 is collected. The fractions with the highest concentrations of active IL-4 based on the conventional SDS-PAGE and protein assays are pooled.

In step (d) the pH of the pooled fractions from step (c) are adjusted to pH 6.75 and diluted with a 20 mM buffer at pH 6.75 so the conductivity becomes 15mS. The cation exchange gel material in a chromatography column is equilibrated with a 20 mM phosphate buffer at pH 6.75 having 0.12 M sodium chloride. A preferred cation exchange is crosslinked agarose substituted with -CH₂-SO₃- Na⁺ groups, such as S-Sepharose® Fast Flow available from Pharmacia. The active IL-4 is gradient eluted with a 20 mM phosphate buffer at pH 6.75 with 0.12-0.6 M NaCI. The fractions with highest concentrations of active IL-4 based on the SDS-PAGE and protein assays are pooled.The conditions for cation exchange chromatography are selected to insure that the active IL-4 fraction will attach to the cation exchanger matrix. The near neutral pH 6.75 which is relatively high for a cation exchange chromatography and the 15mS conductivity which is relatively high for ion exchange chromatography results in mild binding conditions where most impurities do not bind to the column, elution is relatively easy and high purity of the active IL-4 solution is obtained, i.e. about 90% -95%.

In step (e) the pooled eluted fractions from step (d) are concentrated on a stirred cell fitted with a membrane which holds all material with greater than 10,000 molecular weight. This includes active IL-4. Since, at this stage of the process the active IL-4 solution is about 90-95% pure, very little except active IL-4 is in the concentrated solution retained on top of the membrane. A preferred membrane is YM-10 manufactured by Amicon Co., U.S.A. The concentration obtained is up to about 20mg/mL of active IL-4.

In step (f) the pooled, concentrated eluates of active IL-4 from step (e) are charged to a size exclusion gel filtration column which fractionates proteins in the solution according to molecular weight. A typical column which is suitable is a Sephacryl®S-200 HR or S-100HR(Pharmacia) gel filtration column. The Sephacryl®S-200HR(high (high resolution) and S-100HR are crosslinked copolymers of allyldextran and N, N'-methylene bisacrylamide. Their fractionation range in Daltons is 5,000 - 250,000 and 1,000 - 100,000, respectively. Other suitable materials are the Sephadexes® (Pharmacia) which are crosslinked dextran gels. Preferably the solution of active IL-4 is charged to an S-200 HR column previously equilibrated with a 10 mM citrate buffer at pH 4.5. Under the conditions of step (g) the stable IL-4 concentrate can reach up to 20 mg/ml. This increases the capacity and performance of the gel filtration chromatography.

The fractions of eluate containing the highest concentrations of active IL-4 as determined by the SDS-PAGE and protein assay are collected and pooled to result in a 95-99% pure solution of active IL-4.

The buffers utilized in this process are chosen because they provide the proper conditions of binding, washing and elution to enable the active IL-4 either to adsorb to the chromatography gels or elute selectively therethrough. The preferred buffers are sodium phosphate buffers at a concentration of 20 mM or sodium citrate or sodium acetate buffers at the concentrations and pHs as shown in the examples, as well as the specific amount of sodium chloride indicated. The pH is adjusted with sodium hydroxide or acid. In the two part wash of step (b), the first wash is with a20mM sodium phosphate equilibration buffer at pH 7.2 containing 1.0M sodium chloride. and the second wash is with the phosphate buffer containing about 150mM sodium chloride and10% glycerol which is required in the second wash buffer.

The concentration of the sodium chloride in the buffers used with the zinc chelating-Sepharose columns is critical to this invention because high salt, preferably 1M sodium chloride, improves the recovery of solubilized active IL-4 from a trichloroacetic acid (TCA) pellet as well as enhances active IL-4 binding to the metal chelating Sepharose. The concentration enables the IL-4 to be more selectively adsorbed to the zinc chelating Sepharose column than impurities in the fermentation broth.

The bulk IL-4 was then prepared for injection by thawing and diluted with sterilized water and/or 10 mm citrate buffer.

### Example 1

### PREPARATION OF S-SEPHAROSE® FAST FLOW COLUMN

Prepare two cation exchange columns with S-Sepharose® Fast Flow cation exchange resin in a buffer of 20mM sodium phosphate, pH 7.2, and 0.12M NaCl. The first column is a 1.5L column, 100 mm in diameter and 45 cm in height and the second column is a 0.25L column, 50 mm in diameter and 300 mm in height. The smaller column has 15% of the bed volume of the larger column when each column is fully charged with the cation exchange gel. The columns are loaded as follows: Slurry S-Sepharose® Fast Flow gel cation exchange resin in a buffer composed of 20mM sodium phosphate, pH 7.2, ,and 0.12M NaCl. Pour the gel into the appropriate chromatography column, allow the liquid to flow or pump it through the bottom of the column.

Place a top flow adapter on the column and equilibrate the gel with 5 bed volumes of a buffer composed of 20 mM sodium phosphate, pH 7.2, 0.12M NaCl, by pumping the buffer through the column at a linear velocity of approximately 1 cm/min. Adjust the top flow adapter to press firmly on top of the resin bed. Then pump at least 5 bed volumes of a buffer composed of 20 mM sodium phosphate, pH 7.2, 0.12M NaCl, through the column at a linear velocity of approximately 1 cm/min and if necessary continue pumping the buffer through the column at the same flow rate until, the pH of the effluent is between 7.1 and 7.3. Adjust the bed volumes of the columns so the smaller column has 15% the bed volume of the larger column.

### Example 2

### PREPARATION OF COBALT CHELATING-SEPHAROSE® FAST FLOW

Suspend cheating Sepharose®Fast Flow gel in 5 volumes of 0.02 M cobalt acetate and let stand overnight with occasional stirring. Wash the gel on a Buchner funnel with deionized water, then wash the gel with a buffer of 0.02M sodium phosphate, pH 7.2, containing 0.5 M NaCl. Then slurry the gel in 0.5 gel volumes of 0.02 M sodium phosphate buffer at pH 7.2, containing 0.5 M NaCl. Pour 190 mL of the cobalt charged gel into a column 50 mm in diameter and 300 mm in height and pump the liquid through the column. Place a top flow adapter on the column and equilibrate the gel by pumping 0.02 M sodium phosphate buffer at pH 7.2, containing 0.5 M NaCl through the column at approximately 1 cm/min. Place a flow adapter on top of the column.

### Example 3

### PREPARATION OF CHELATING SEPHAROSE®FAST FLOW (NOT TREATED WITH A METAL SALT)

Slurry Cheating Sepharose®Fast Flow gel in a buffer composed of 20mM sodium phosphate at pH7.2 with 0.5 M NaCl. Pour 75 mL of the gel into the chromatography column from Example 2 so that the gel not treated with the metal salt settles in a uniform layer on the top of the cobalt charged gel. Place a flow adaptor on the column and equilibrate the gel with 0.02M sodium phosphate buffer at pH 7.2 containing 0.5 M NaCl.

### Example 4

### EQUILIBRATION OF COBALT CHELATING-SEPHAROSE® COLUMN

After the cobalt cheating Sepharose® Fast Flow and the untreated cheating Sepharose®column is prepared according to Examples 2 and 3, invert the column so the cobalt treated resin is on top of the untreated resin. Continue pumping the buffer, i.e. 0.02 M sodium phosphate, pH 7.2, 0.5 M NaCl, through the column until the pH of the effluent is between 7.1 and 7.3.

### Example 5

### PREPARATION OF SEPHACRYL®S-200 HR COLUMN

Pump at least 1 bed volume of a buffer composed of 10 mM sodium citrate, pH 4.5, through the S-200 HR gel in a 1.8L chromatography column, 50 mm diameter, 100 cm height, at a linear velocity of approximately 0.2 cm/min to equilibrate the column.

### Example 6

### PREPARATION OF BUFFERS

(a) 0.02M Sodium Phosphate, pH 7.2, 0.12 M Sodium Chloride
   Mix together in deionized water 2.78 g/L sodium phosphate monobasic monohydrate, 7.03 g/L sodium chloride and sufficient amount of 6.3N sodium hydroxide to adjust the pH to 7.2(±0.1).
(b) 0.02M Sodium Phosphate, pH 7.2, 0.26M Sodium Chloride.
   Mix together in deionized water 2.78 g/L sodium phosphate monobasic monohydrate and 15.19 g/L sodium chloride. Adjust the pH to 7.2 (±0.1) with 6.3N sodium hydroxide.
(c) 0.02M Sodium Phosphate, pH 7.2, 0.50 M Sodium Chloride
   Mix together in deionized water 2.78 g/L sodium phosphate monobasic monohydrate, 29.22 g/L sodium chloride and sufficient amount of 50% NaOH to adjust the pH to 7.2(±0.1).
(d) 0.02M Sodium Phosphate, pH 6.0, 0.05M EDTA, 0.5 M Sodium Chloride
   Mix together in deionized water 2.78 gm/L sodium phosphate monobasic monohydrate and 19 g/L tetrasodium EDTA and 29.22 g/L sodium chloride. Adjust the pH to 6.0 (±0.1) with 6.3N sodium hydroxide/4N HCl.
(e) 10mM Sodium Citrate, pH 4.5
   Mix 210 grams (2.1 g/L) citric acid monohydrate with 100L deionized water until the citric acid monohydrate is dissolved. Adjust the pH of the buffer to 4.5 with 4N hydrochloric acid and 6.3N sodium hydroxide, if needed. This buffer is used for diafiltration and ultrafiltration as well as for gel filtration chromatography.
(f) 20 mM Sodium Phosphate, pH 7.2
   Mix 278 grams sodium phosphate monobasic monohydrate with 100L deionized water and agitate until dissolved. Adjust the pH of the buffer to pH 7.2 with 50% NaOH and to a conductivity of 2-4mS.

### Example 7

### CATION EXCHANGE CHROMATOGRAPHY TREATMENT OF CRUDE IL-4 SOLUTION

Adjust the crude IL-4 containing cell culture medium (total protein about 14,000g) to pH 7.2 (^{±}0.1) and adjust its conductivity to 14 mS(±1). Pump the solution through the S-Sepharose® cation exchange column at a linear velocity of approximately 1.0 cm/min or less. Wash the column with the buffer prepared in Example 6(a). Isocratically elute the column with the buffer prepared in Example 6(b) at a linear velocity of approximately 0.2 cm/min. Collect the fractions which contain active IL-4 as determined by the SDS-PAGE and protein assays and pool them. The purity of the pooled active IL-4 is about 50%.

### Example 8

### GRADIENT ELUTION OF PARTIALLY PURIFIED ACTIVE IL-4 SOLUTION ON CATION EXCHANGE COLUMN

Adjust the pH of the pooled active IL-4 solution made according to Example 7 to 7.2 (±0.1) with either 4NHCl or 6.3N NaOH as required. Adjust the conductivity of the solution with the buffer prepared in Example 6(f) to 14mS(± 1).

Pump the solution through an S-Sepharose® column as prepared in Example 1 at a linear velocity of approximately 1 cm/min or less. Collect the flow-through solution in one fraction.

Elute the column using a gradient of approximately 1.75 mS per bed volume and a linear flow rate of approximately 0.2 cm/min.

The low salt buffer used in the gradient is that made in Example 6(a), and the high salt buffer used in the gradient is that made in Example 6(c).

Collect 5 large fractions, each with a volume of approximately 0.5 bed volumes. Collect the remaining fractions (about 40-50) in volumes of 0.1 bed volumes.

Analyze the fractions for active IL-4 by the SDS-PAGE and protein assays. Pool the active IL-4 fractions. The purity of the pooled active IL-4 solution is about 60% to 70%.

### Example 9

### COBALT CHELATING SEPHAROSE® CHROMATOGRAPHY PURIFICATION OF ACTIVE RECOMBINANT HUMAN INTERLEUKIN-4

Adjust the pH of the IL-4 solution from Example 8 to 7.2 (±0.1) with 4N HCl and/or 6.3N sodium chloride. Adjust the conductivity of the solution to 45-50 mS.

Clarify the solution if necessary by centrifugation or microfiltration. Pump the solution (about 3.3 mg protein per mL gel) through the cobalt chelating Sepharose®Fast Flow and untreated chelating Sepharose® Fast Flow columns as prepared in Example 4 at a linear velocity of approximately 0.5 cm/min. Collect the flow-through in one fraction.

Wash the columns with approximately 10 bed volumes of the buffer prepared in Example 6(c) at a linear velocity of approximately 0.5 cm/min and collect the wash in no more than 5 fractions, then elute the column with approximately 10 bed volumes of the buffer made in Example 6(d) at a linear velocity of approximately 0.5 cm/min. Collect fractions with a volume of approximately 0.2 bed volumes. Analyze each sample for active IL-4 with SDS-PAGE and protein assays and pool the active IL-4 fractions..

The purity of the active IL-4 solution treated according to this Example 9 is about 90 - 95%.

### Example 10

### ULTRAFILTRATION AND CONCENTRATION

Concentrate the pooled fractions from Example 9 using an Amicon stirred chamber fitted with a YM-10 membrane by placing the pooled fractions from Example 9 containing active human IL-4 in a container and adding approximately 0.25 volumes of the buffer prepared in Example 6(d). Concentrate the volume to approximately 0.2 the original volume by ultrafiltration on the YM-10 membrane. Dilute the concentrated retentate with 4 volumes of the buffer prepared in Example 6(e) and concentrate it to approximately 0.2 volumes by ultrafiltration on the YM-10 membrane.

The concentration step can be repeated to achieve approximately 0.1 the volume of the initial pooled fractions. Transfer the concentrate to an appropriate container and hold at cold room temperature for immediate use or store frozen at -20°C.

### Example 11

### GEL FILTRATION CHROMATOGRAPHY (SIZE EXCLUSION)

Equilibrate a Sephacryl® S-200 HR column with the buffer prepared in Example 6(e) by pumping at least one bed volume of the buffer through the column at a linear velocity of approximately 0.2 cm/min.

Clarify the solution made in Example 10 by centrifugation on a laboratory centrifuge at 4500 rpm for 30 minutes at 2°C-6°C. Measure the Å₂₈₀ and dilute the solution with the buffer prepared in Example 6(e) so there are 5 optical density units at 280 nm per mL.

Pump the resulting solution onto a Sephacryl® S-200 HR column at a linear velocity of approximately 0.1 cm/min. Continue pumping the buffered of Example 6(e) through the column at a flow rate of approximately 0.1 cm/min. Collect one large fraction having a total volume of 0.4 to 0.55 bed volume, then collect 50 fractions of approximately 0.01 bed volume.

Select the fractions with the active IL-4 as determined by SDS-PAGE and protein assay. Pool the active IL-4 fractions and fitter through a 0.2 micron sterile filter. Recover the filtrates which are 95% to 99% pure active IL-4 solutions as evidenced by the SDS-PAGE assay.. The overall yield based on the active IL-4 in the cell culture medium is 70%.

All the assays used to determine the active fractions of interleukin-4 are conventional. For UV absorbance at 280nm measurement of purified IL-4, 1.0 A₂₈₀ Optical Density Unit (OD) is equivalent to 1.6 mg by amino acid composition analysis and to 2.0 mg by Lowry's Method.

### Example 12

### PREPARATION OF ZINC CHELATING-SEPHAROSE® FAST FLOW

Slurry chelating Sepharose®Fast Flow gel in deionized water. To prepare a 3 liter column, pour the slurry into a chromatography column 500 mm high with a diameter of 180 mm. Allow the liquid in the column to flow or pump it through the bottom of the column.

Place a top flow adaptor on the column and pack/wash the gel with deionized water. Pump the water through the column at a linear velocity of approximately 1 cm/ min. Adjust the top flow adaptor to press firmly on top of the resin bed. Pump at least 5 bed volumes of deionized water through the columns at a linear velocity of approximately 1 cm/ min.

Pump approximately 5 bed volumes of 0.023 M zinc acetate solution through the column at a linear velocity of approximately 1 cm/minute. Pump approximately 10 bed volumes of deionized water through the column at a linear velocity of approximately 1 cm/ min.

Continue pumping the phosphate buffer solution through the column at a linear velocity of approximately 1.0 cm/min. until the pH of the effluent is between 7.1-7.3

### Example 13

### PREPARATION OF CHELATING SEPHAROSE®FAST FLOW (NOT TREATED WITH A METAL SALT)

Slurry Cheating Sepharose®Fast Flow gel in a buffer composed of 20mM sodium phosphate at pH7.2 with 1.0M NaCl. To prepare a 1 liter column pour the gel into a chromatography column of 140 mm diameter and 500 mm height and elute from the bottom of the column. Equilibrate the gel with approximately 5 bed volumes of a buffer composed of 20 mM sodium phosphate at pH 7.2 with 1.0M NaCl. Pump the buffer through the column at a linear velocity of approximately 1 cm/min. Continue pumping the buffer through the column at the same flow rate until the pH of the effluent is between 7.1 and 7.3.

### Example 14

### PREPARATION OF COLUMNS IN SERIES

After the zinc chelating Sepharose® Fast Flow and the untreated chelating Sepharose®columns are prepared according to Examples 12 and 13, connect the columns in series so the flow is first into the zinc chelating column and then into the non-zinc (untreated) chelating column. A bubble trap should not be inserted between the columns.

### Example 15

### PREPARATION OF S-SEPHAROSE®COLUMN

Slurry S-Sepharose gel cation exchange resin in a buffer composed of 20mM sodium phosphate, pH 6.75, 0.12M NaCl and 0.001M ethylene diamine tetraacetic acid (EDTA). Pour the gel into a chromatography column, 100 mm diameter, 45 cm height and allow the liquid to flow or pump it through the bottom of the column.

Place a top flow adapter on the column and equilibrate the gel with 5 bed volumes of a buffer composed of 20 mM sodium phosphate, pH 6.75, 0.12M NaCl, 0.001M EDTA, by pumping the buffer through the column at a linear velocity of approximately 1 cm/min. Adjust the top flow adapter to press firmly on top of the resin bed. Then pump at least 5 bed volumes of a buffer composed of 20 mM sodium phosphate, pH 6.75, 0.12M NaCl, 0.001M EDTA through the column at a linear velocity of approximately 1 cm/min and if necessary continue pumping the buffer through the column at the same flow rate until the pH of the effluent is between 6.6 and 6.9.

### Example 16

### PREPARATION OF SEPHACRYL®S-200HR COLUMN

Pump at least 1 bed volume of a buffer composed of 10 mM sodium citrate, pH 4.5, through the S 200 HR gel in a chromatography column, 50 mm diameter, 100 cm height, at a linear velocity of approximately 0.2 cm/min to equilibrate the column.

### Example 17

### PREPARATION OF BUFFERS

(a) 0.02M Sodium Phosphate, pH 7.2, 1.0M Sodium Chloride
   Mix together in deionized water 2.78 g/L sodium phosphate monobasic monhydrate, 58.44 g/L sodium chloride and sufficient amount of 6.3N sodium hydroxide to adjust the pH to 7.2(±0.1). The batch should be large enough to provide at least 30 liters per liter of the gels in the metal chelating Sepharose® columns.
(b) 0.02M Sodium Phosphate, pH 7.2, 0.15M Sodium Chloride, 10% Glycerol
   Mix together in deionized water 2.78 g/L sodium phosphate monobasic monohydrate and 8.76 g/L sodium chloride. Adjust the pH to 7.2 (±0.1) with 6.3N sodium hydroxide. Add sufficient glycerol to provide 0.1 L/L. Prepare sufficient amount of the buffer to provide at least 6 liters per liter of the gel with which it will be used.
(c) 0.02M Sodium Acetate, pH 5.0, 0.5M Sodium Chloride
   Mix together in deionized water 1.15 ml/L of 99.7% acetic acid and 29.2 g/L sodium chloride. Adjust the pH to 5.0 (±0.1) with 6.3N sodium hydroxide. Prepare sufficient amount of the buffer to provide at least 10 liters per liter of the gel with which it will be used.
(d) 0.03M Sodium Phosphate, pH 7.0, 0.003M EDTA
   Mix together in deionized water 4.17 gm/L sodium phosphate monobasic monohydrate and 1.14 g/L tetrasodium EDTA. Adjust the pH to 7.0 (±0.1) with 6.3N sodium hydroxide. Prepare sufficient amount of the buffer to provide at least 2 liters per liter of gel
(e) 10mM Sodium Citrate, pH 4.5
   Mix 210 grams (2.1 g/L) citric acid monohydrate with 100L deionized water until the citric acid monohydrate is dissolved. Adjust the pH of the buffer to 4.5 with 4N hydrochloric acid and 6.3N sodium hydroxide, if needed. This buffer is used for diafiltration and ultrafiltration as well as for gel filtration chromatography.

### Example 18

### PURIFICATION OF ACTIVE RECOMBINANT HUMAN INTERLEUKIN-4

### (Zinc chelating Sepharose® chromatography)

Concentrate 700 liters of a fermentation broth in which active human recombinant IL-4 is dissolved to 20 liters, then subject the solution to a 25-fold diafiltration against the buffer of Example 17(a). Adjust the pH of the solution to 7.2 (±0.1) with 4N HCl and/or 6.3N sodium chloride. Adjust the conductivity of the solution to 70-90 mS.

Clarify and concentrate the solution by filtration. Wash the filter with the buffer of Example 17(a) to return the volume of the solution to 20 liters.

Pump the solution through the zinc chelating Sepharose®Fast Flow and untreated Sepharose® Fast Flow columns at a linear velocity of approximately 0.5 cm/min. Collect the flow-through in one fraction.

The columns must be washed twice, first with approximately 10 bed volumes of the buffer prepared in Example 17(a) at a linear velocity of approximately 0.5 cm/min and collect the wash in no more than 5 fractions, then with approximately 5 bed volumes of the buffer made in Example 17(b) at a linear velocity of approximately 0.5 cm/min and collect the wash in 1 fraction.

Elute the active IL-4 from the column with approximately 8 bed volumes of the buffer made in Example 17(c) at a linear velocity of approximately 0.25 cm/min. Collect fractions with a volume of approximately 0.2 bed volumes in separate containers containing as a diluent 0.5 ml per ml to be collected of the buffer made in Example 17(d).

Test each sample for active IL-4 with SDS-PAGE and protein assays.

The purity of the active IL-4 solution treated according to this Example 18 is about 20%-40%. The yield of active IL-4 based on the amount in the crude fermentation broth is 90%.

### Example 19

### PREPARATION OF BUFFERS FOR S-SEPHAROSE® CHROMATOGRAPHY

(a) 20 mM Sodium Phosphate, pH 6.75, 0.12M Sodium Chloride, 0.001M EDTA
   Charge 2.78 g/L sodium phosphate monobasic monohydrate, 7.03 g/L sodium chloride, 0.38 g/L tetra sodium EDTA and 1 L/L of deionized water into an appropriate container and agitate until dissolved.
   Adjust the pH of the buffer solution to 6.75 (±0.1) with 6.3N sodium hydroxide.
(b) 20mM Sodium Phosphate, pH 6.75 0.55M Sodium Chloride, 0.001M EDTA
   Charge 2.78 g/L. sodium phosphate monobasic monohydrate, 32.14 g/L sodium chloride, 0.38 g/L tetra sodium EDTA and deionized water into an appropriate container and agitate until dissolved. Adjust the pH of the buffer solution to 6.75 (±0.1) with 6.3N sodium hydroxide.
(c) 20mM Sodium Phosphate, pH 6.75, 0.001M EDTA
   Charge into a container sufficiently large to hold 2.78 g/L sodium phosphate monobasic monohydrate, 0.38g/L tetra sodium EDTA and deionized water. Agitate until dissolved and adjust the pH of the buffer to 6.75 (±0.1) with 6.3N Na OH as required.

### Example 20

### GRADIENT ELUTION OF PURIFIED ACTIVE INTERLEUKIN-4 SOLUTION ON CATION EXCHANGE COLUMN.

Adjust the pH of the active IL-4 solution made according to Example 7 to 6.75 (±0.1) with either 4NHCl or 6.3N NaOH as required.

Filter through a 0.45 micron filter. Wash the filter with approximately 1 liter of the buffer prepared in Example 19(c).

Adjust the conductivity of the resulting solution to 15 (± 0.5 mS) with the buffer solution prepared in Example 19(c)

Pump the solution through an S-Sepharose® column as prepared in Example 4 at a linear velocity of approximately 1cm/min or less. Collect the effluent solution in one fraction.

Wash the column with approximately 5 bed volumes of the buffer solution prepared in Example 19(a) at a linear velocity of approximately 1.0 cm/min or less.

Collect the wash in 1 fraction. Elute the column using a gradient of approximately 4.5 mS per bed volume and a linear flow rate of approximately 0.2 cm/min.

The low salt buffer used in the gradient is that made in Example 19(a), at 5 bed volume and the high salt buffer used in the gradient is that made in Example 19(b), at 5 bed volume.

Collect 5 large fractions, each with a volume of approximately 0.8 bed volumes.

Collect the remaining fractions (about 40-50) in volumes of 0.1 bed volumes.

Test the fractions for active IL-4 by the SDS-PAGE and protein assays.

Pool the active IL-4 fractions. The yield of active IL-4 based on the amount of active IL-4 in the zinc cheating pooled eluates is 90%.

The purity of the active IL-4 solution is about 90% to 95%.

### Example 21

### ULTRAFILTRATION AND CONCENTRATION

Concentrate the pooled fractions from Example 20 using an Amicon stirred chamber fitted with a YM 10 membrane by placing the pooled fractions from Example 20 containing active human IL-4 in a container and concentrating the volume to approximately 0.1 the original volume by ultrafiltration on a YM-10 membrane. Dilute the concentrated retentate with 4 volumes of the buffer prepared in Example 17(e) and concentrate it to approximately 0.2 volumes by ultrafiltration on a YM-10 membrane.

The concentration step can be repeated to achieve approximately 0.1 the volume of the initial pooled fractions. Transfer the concentrate to an appropriate container and hold at cold room temperature or store frozen at 20°C.

### Example 22

### GEL FILTRATION (SIZE EXCLUSION)

Clarify the solution made in Example 21 by centrifugation on a laboratory centrifuge at 4500 rpm for 30 minutes at 2°C-6°C. Measure the Å₂₈₀ and dilute the solution with the buffer prepared in Example 17(e) so there are 15Å₂₈₀/ml.

Pump the resulting solution onto a Sephacryl® S-200 HR column at a linear velocity of approximately 0.1 cm/min. Continue pumping the buffer of Example 17(e) through the column at a flow rate of approximately 0.1 cm/min. Collect five fractions having a total volume of 0.4 to 0.55 bed volume, then collect 50 fractions of approximately 0.01 bed volume.

Select the fractions with the active IL-4 as determined by SDS-PAGE and protein assay. Pool the active IL-4 fractions and filter through a 0.2 micron sterile filter. Recover the filtrates which are 95% to 99% pure active IL-4 solutions. The overall yield based on the active IL-4 in the fermentation broth is 70-80%.

The purity of the recovered active IL-4 is 95-99% as evidenced by the SDS-PAGE assay.

All the assays used to determine the active fractions of interleukin-4 are conventional. The assays required for selecting active fractions from the eluates are the UV absorbance at 280nm measurement. For purified IL-4, 1.0Å₂₈₀ Optical Density Unit (OD) is equivalent to 1.6 mg by amino acid composition analysis and to 2.0 mg by Lowry's Method.

SDS-PAGE assay is also required for selecting active fractions. This method is discussed in Laemmli, U. K., Nature, 227 : 680 (1970).

Lowry's Method is described in Lowry et al., J. Biol. Chem.

While the present invention has been described in connection with certain specific embodiments, it will be evident to one of ordinary skill in the art that many alternatives, modifications and variations may be made. All such alternatives, modifications and variations are intended to be included within the spirit and scope of the invention.

SEQUENCE LISTING

SEQ ID NO: 1
SEQUENCE TYPE: Amino acid
SEQUENCE LENGTH: 129 amino acids
PROPERTIES: Human interleukin-4


1               5                   10                  15

                20                  25                  30

                35                  40                  45

                50                  55                  60

                65                  70                  75

                80                  85                  90

                95                  100                 105

                110                 115                 120

                125

## Claims

1. A process for purifying a crude solution of active recombinant human interleukin-4, comprising
(a) Charging said crude solution of IL-4 buffered at a neutral to slightly alkaline pH and containing about 0.5-1.5M sodium chloride to a metal cheating agarose gel chromatography column to selectively bind the active recombinant human interleukin-4 to the column;
(b) washing said column twice, first with an equilibration buffer containing 1.0M sodium chloride and then with a buffer containing 10% glycerol and 0.15M sodium chloride;
(c) eluting the bound active recombinant human interleukin-4 from the column with an eluting buffer at about pH 4.5 to 5.5;
(d) charging the active human IL-4 solution from (c) in a buffer to chromatography on a cation exchanger and gradient eluting a buffered solution of the active human IL-4 from the column;
(e) concentrating the eluate from (d) on a stirred cell membrane that allows matter of less than 10,000 molecular weight to pass;
(f) subjecting the concentrate from (e) to size exclusion chromatography ; and
(g) recovering the purified solution of active recombinant human interleukin-4.

2. The process of Claim 1 wherein in step (a) the buffer is a phosphate buffer of pH 7.5 and the sodium chloride concentration is 1M. and the metal chelating gel column is a zinc chelating agarose gel.

3. The process of Claim 1 or Claim 2 wherein in step (b) the buffer is a 0.02 mM sodium phosphate at pH 7.2 also having 150mM sodium chloride therein.

4. The process of any of Claims 1 to 3 wherein step (c) the eluting buffer is a 0.02 mM sodium acetate buffer with 0.5mM sodium chloride and pH 5.0.

5. the process of any of Claims 1 to 4 wherein the step (d) the charging buffer is a 20 mM phosphate buffer at pH 6.75 with 0.12M sodium chloride and the eluting buffer is a 20 mM phosphate buffer at pH 6.75 with about 0.12-0.55M sodium chloride.

6. The process of any of Claims 1 to 5 wherein the concentration of step (e) is accomplished by diafiltration against 10 mM sodium citrate buffer at pH 4.5 to a concentration of up to 20 mg/mL.

7. A process for purifying a crude solution of active recombinant human interleukin-4 expressed from CHO-cell lines, comprising
(a) charging said crude solution of active IL-4 buffered at a neutral to slightly alkaline pH and 13 to 15 mS to cation exchange chromatography on a cross-linked agarose gel matrix column to selectively bind the active recombinant human interleukin-4 to the column, washing with an equilibration buffer and isocratically eluting the active IL-4 from the column;
(b) charging the active human IL-4 solution from (a) in a buffer to chromatography on a smaller cation exchange column having about 15% the bed volume of the cation exchange column in (a), washing with an equilibration buffer, gradient eluting the bound active IL-4 from the column with a buffer system at pH 7.2, containing 0.12 - 0.50M sodium chloride and pooling the active eluates;
(c) adjusting the pH of the eluate pool to pH 7.2 and the conductivity to 45 - 50mS, then charging the pooled eluates to a metal chelating agarose gel column in a buffer at about pH 6.7 to 8 and containing about 0.5M sodium chloride, then washing the column with a buffer at near neutral pH containing 0.5M sodium chloride, then isocratically eluting the active IL-4 with a buffer at pH 6.0 containing 0.50M sodium chloride;
(d) concentrating and diafiltrating the eluate from (c) at pH 4.5 on a stirred cell membrane that allows matter of less than 10,000 molecular weight to pass;
(e) charging the concentrate from (d) to size exclusion chromatography column on a crosslinked coplymer gel of allyldextran and N,N'-methylene bisacrylamide equilibrated with a buffer system at pH 4.5; and
(f) collecting the purified solution of active recombinant human interleukin-4.

8. The process of Claim 7 wherein in step (a) the equilibration buffer is 20 mM sodium phosphate at pH 7.2, containing 0.12M sodium chloride concentration.

9. The process of Claim 7 or Claim 8 wherein in step (b) the eluting buffer is a 20 mM sodium phosphate at pH 7.2 having 0.12-0.50M sodium chloride gradient therein and the metal chelating gel column is a cobalt chelating agarose gel.

10. The process of any of Claims 7 to 9 wherein in step (c) the eluting buffer is a 20 mM phosphate buffer at pH 6.0 containing a 0.50M sodium chloride.

11. The process of any of Claims 7 to 10 wherein the concentration step (d) is accomplished by diafiltration against 10mM sodium citrate buffer at pH 4.5 to a concentration of up to 20mg/mL.

12. The processes of any of Claims 7 to 11 wherein in step (e) the column is equilibrated with 10 mM sodium citrate.

## Patentansprüche

1. Verfahren zur Reinigung einer rohen Lösung von aktivem rekombinantem Human-Interleukin-4, umfassend:
(a) Einfüllen der rohen IL-4-Lösung, die auf einen neutralen bis leicht alkalischen pH-Wert gepuffert ist und etwa 0,5-1,5 M Natriumchlorid enthält, in eine Chromatographiesäule mit metallchelatisierendem Agarosegel, so daß das aktive rekombinante Human-Interleukin-4 selektiv an die Säule gebunden wird;
(b) zweimaliges Waschen der Säule, zuerst mit einem Äquilibrierungspuffer, der 1,0 M Natriumchlorid enthält, und dann mit einem Puffer, der 10% Glycerin und 0,15 M Natriumchlorid enthält;
(c) Eluieren des gebundenen aktiven rekombinanten Human-Interleukin-4 von der Säule mit einem Elutionspuffer mit einem pH-Wert von etwa 4,5 bis 5,5;
(d) Einfüllen der aktiven Human-IL-4-Lösung aus (c) in einem Puffer zur Chromatographie auf einem Kationenaustauscher und Eluieren einer gepufferten Lösung des aktiven Human-IL-4 in einem Gradienten von der Säule;
(e) Konzentrieren des Eluats aus (d) auf einer Rührzellmembran, die Substanzen mit einem Molekulargewicht von weniger als 10 000 durchläßt;
(f) Durchführen einer Ausschlußchromatographie mit dem Konzentrat aus (e); und
(g) Gewinnen der gereinigten Lösung von aktivem rekombinantem Human-Interleukin-4.

2. Verfahren gemäß Anspruch 1, wobei der Puffer in Schritt (a) ein Phosphatpuffer mit dem pH-Wert 7,5 ist und die Natriumchloridkonzentration 1 M beträgt und es sich bei der Säule mit dem metallchelatisierenden Gel um ein zinkchelatisierendes Agarosegel handelt.

3. Verfahren gemäß Anspruch 1 oder 2, wobei der Puffer in Schritt (b) 0,02 mM Natriumphosphat mit dem pH-Wert 7,2 ist und auch 150 mM Natriumchlorid enthält.

4. Verfahren gemäß einem der Ansprüche 1 bis 3, wobei der Elutionspuffer in Schritt (c) ein 0,02 mM Natriumacetatpuffer mit 0,5 mM Natriumchlorid und dem pH-Wert 5,0 ist.

5. Verfahren gemäß einem der Ansprüche 1 bis 4, wobei in Schritt (d) der Puffer beim Einfüllen ein 20 mM Phosphatpuffer mit dem pH-Wert 6,75 und 0,12 M Natriumchlorid ist und der Elutionspuffer ein 20 mM Phosphatpuffer mit dem pH-Wert 6,75 und etwa 0,12-0,55 M Natriumchlorid ist.

6. Verfahren gemäß einem der Ansprüche 1 bis 5, wobei das Konzentrieren in Schritt (e) durch Diafiltration gegen 10 mM Natriumcitratpuffer bei pH 4,5 auf eine Konzentration von bis zu 20 mg/ml erfolgt.

7. Verfahren zur Reinigung einer rohen Lösung von aktivem rekombinantem Human-Interleukin-4, das von CHO-Zellinien exprimiert wurde, umfassend:
(a) Einfüllen der rohen Lösung von aktivem IL-4, die auf einen neutralen bis leicht alkalischen pH-Wert gepuffert ist und eine Leitfähigkeit von 13 bis 15 mS besitzt, zur Kationenaustausch-Chromatographie auf einer Säule mit einer Matrix aus vernetztem Agarosegel, so daß das aktive rekombinante Human-Interleukin-4 selektiv an die Säule gebunden wird, Waschen mit einem Äquilibrierungspuffer und isokratisches Eluieren des aktiven IL-4 von der Säule;
(b) Einfüllen der aktiven Human-IL-4-Lösung aus (a) in einem Puffer zur Chromatographie auf einer kleineren Kationenaustauschersäule, die etwa 15% des Bettvolumens der Kationenaustauschersäule in (a) besitzt, Waschen mit einem Äquilibrierungspuffer, Eluieren des gebundenen aktiven IL-4 in einem Gradienten von der Säule mit einem Puffersystem mit dem pH-Wert 7,2, das 0,12-0,50 M Natriumchlorid enthält, und Zusammengießen der aktiven Eluate;
(c) Einstellen des pH-Werts der zusammengegossenen Eluate auf pH 7,2 und der Leitfähigkeit auf 45-50 mS, dann Einfüllen der zusammengegossenen Eluate in einem Puffer mit einem pH-Wert von etwa 6,7 bis 8, der etwa 0,5 M Natriumchlorid enthält, in eine Säule mit metallchelatisierendem Agarosegel, dann Waschen der Säule mit einem Puffer mit ungefähr neutralem pH-Wert, der 0,5 M Natriumchlorid enthält, dann isokratisches Eluieren des aktiven IL-4 mit einem Puffer mit dem pH-Wert 6,0, der 0,50 M Natriumchlorid enthält;
(d) Konzentrieren und Diafiltrieren des Eluats aus (c) bei einem pH-Wert von 4,5 auf einer Rührzellmembran, die Substanzen mit einem Molekulargewicht von weniger als 10 000 durchläßt;
(e) Einfüllen des Konzentrats aus (d) in eine Säule zur Ausschlußchromatographie auf einem vernetzten Copolymergel aus Allyldextran und N,N'-Methylenbisacrylamid, das mit einem Puffersystem auf pH 4,5 äquilibriert ist; und
(f) Auffangen der gereinigten Lösung von aktivem rekombinantem Human-Interleukin-4.

8. Verfahren gemäß Anspruch 7, wobei der Äquilibrierungspuffer in Schritt (a) 20 mM Natriumphosphat mit dem pH-Wert 7,2 ist und eine Natriumchloridkonzentration von 0,12 M aufweist.

9. Verfahren gemäß Anspruch 7 oder 8, wobei der Elutionspuffer in Schritt (b) 20 mM Natriumphosphat mit dem pH-Wert 7,2 ist und einen Natriumchloridgradienten von 0,12-0,50 M enthält und es sich bei der Säule mit dem metallchelatisierenden Gel um ein cobaltchelatisierendes Agarosegel handelt.

10. Verfahren gemäß einem der Ansprüche 7 bis 9, wobei der Elutionspuffer in Schritt (c) 20 mM Phosphatpuffer mit dem pH-Wert 6,0 ist, der 0,50 M Natriumchlorid enthält.

11. Verfahren gemäß einem der Ansprüche 7 bis 10, wobei der Schritt des Konzentrierens (d) durch Diafiltration gegen 10 mM Natriumcitratpuffer bei pH 4,5 auf eine Konzentration von bis zu 20 mg/ml erfolgt.

12. Verfahren gemäß einem der Ansprüche 7 bis 11, wobei die Säule in Schritt (e) mit 10 mM Natriumcitrat äquilibriert wird.

## Revendications

1. Procédé de purification d'une solution brute d'interleukine-4 humaine recombinante active consistant à
(a) charger ladite solution brute de IL-4 tamponnée à un pH neutre à légèrement alcalin et contenant du chlorure de sodium à environ 0,5-1,5M dans une colonne de chromatographie sur gel agarose complexant les métaux pour lier sélectivement l'interleukine-4 humaine recombinante active à la colonne,
(b) laver deux fois ladite colonne, d'abord avec un tampon d'équilibrage contenant du chlorure de sodium 1,0M puis avec un tampon contenant 10% de glycérol et du chlorure de sodium 0,15M ;
(c) éluer l'interleukine-4 humaine recombinante active liée de la colonne avec un tampon d'élution à un pH d'environ 4,5 à 5,5 ;
(d) charger la solution de IL-4 humaine active de (c) dans un tampon pour chromatographier sur un échangeur de cations et éluer avec gradient une solution tamponnée de IL-4 humaine active de la colonne ;
(e) concentrer l'éluat de (d) sur une membrane cellulaire sous agitation qui permet le passage d'une matière d'un poids moléculaire de moins de 10 000 ;
(f) soumettre le concentré de (e) à une chromatographie par exclusion de taille ; et
(g) récupérer la solution purifiée de l'interleukine-4 humaine recombinante active.

2. Procédé de la revendication 1 où à l'étape (a) le tampon est un tampon de phosphore à pH 7,5 et la concentration du chlorure de sodium est de 1M et la colonne de gel complexant les métaux est un gel d'agarose complexant le zinc.

3. Procédé de la revendication 1 ou de la revendication 2 où à l'étape (b) le tampon est un phosphate de sodium 0,02 mM à pH 7,2 contenant également du chlorure de sodium 150 mM.

4. Procédé selon l'une des revendications 1 à 3 où à l'étape (c) le tampon d'élution est un tampon d'acétate de sodium 0,02 mM avec du chlorure de sodium 0,5 mM et pH 5,0.

5. Procédé selon l'une des revendications 1 à 4 où à l'étape (d) le tampon de charge est un tampon de phosphate 20 mM à pH 6,75 avec du chlorure de sodium 0,12 M et le tampon d'élution est un tampon de phosphate 20 mM à pH 6,75 avec du chlorure de sodium à environ 0,12-0,55 M.

6. Procédé selon l'une des revendications 1 à 5 où la concentration de l'étape (e) est accomplie par diafiltration avec un tampon de citrate de sodium 10 mM à un pH de 4,5 jusqu'à une concentration pouvant atteindre 20 mg/ml.

7. Procédé de purification d'une solution brute d'interleukine-4 humaine recombinante active exprimée de lignées de cellules CHO, consistant à
(a) charger ladite solution brute de IL-4 active tamponnée à un pH neutre jusqu'à légèrement alcalin et 13 à 15 mS pour une chromatographie par échange de cations sur une colonne d'une matrice de gel agarose réticulé pour lier sélectivement l'interleukine-4 humaine recombinante active à la colonne, laver avec un tampon d'équilibrage et éluer isocratiquement IL-4 active de la colonne ;
(b) charger la solution de IL-4 humaine active de (a) dans un tampon pour chromatographier sur une plus petite colonne d'échange de cations ayant un volume du lit d'environ 15% de la colonne d'échange de cations de (a), laver avec un tampon d'équilibrage, éluer avec gradient IL-4 active de la colonne avec un système tampon à pH 7,2 contenant du chlorure de sodium 0,12-0,50M et rassember les éluats actifs ;
(c) ajuster le pH de la masse de l'éluat à pH 7,2 et la conductivité à 45-50 mS puis charger les éluats rassemblés dans une colonne de gel d'agarose complexant les métaux dans un tampon à un pH d'environ 6,7 à 8 et contenant du chlorure de sodium aux environs de 0,5M, puis laver la colonne avec un tampon à un pH presque neutre contenant du chlorure de sodium 0,5 M, puis éluer isocratiquement IL-4 active avec un tampon à pH 6,0 contenant du chlorure de sodium 0,50M ;
(d) concentrer et soumettre à une diafiltration l'éluat de (c) à pH 4,5 sur une membrane cellulaire sous agitation qui permet le passage d'une matière d'un poids moléculaire inférieur à 10 000 ;
(e) charger le concentré de (d) dans une colonne de chromatographie par exclusion de taille sur un gel de copolymère réticulé d'allyldextrane et de N,N'-méthylène bisacrylamide équilibré avec un système tampon à pH 4,5 ; et
(f) collecter la solution purifiée de l'interleukine-4 humaine recombinante active.

8. Procédé de la revendication 7 où à l'étape (a) le tampon d'équilibrage est du phosphate de sodium 20 mM à un pH 7,2 contenant une concentration de chlorure de sodium de 0,12M.

9. Procédé de la revendication 7 ou de la revendication 8 où à l'étape (b) le tampon d'élution est un phosphate de sodium 20 mM à pH 7,2 ayant un gradient de chlorure de sodium 0,12-0,50M et la colonne de gel complexant les métaux est un gel d'agarose complexant le cobalt.

10. Procédé selon l'une des revendications 7 à 9 où à l'étape (c) le tampon d'élution est un tampon de phosphate 20 mM à pH 6,0 contenant du chlorure de sodium 0,50M.

11. Procédé selon l'une des revendications 7 à 10 où l'étape de concentration (d) est accomplie par diafiltration avec un tampon de citrate de sodium 10 mM à pH 4,5 jusqu'à une concentration pouvant atteindre 20 mg/ml.

12. Procédé selon l'une des revendications 7 à 11 où à l'étape (e) la colonne est équilibrée avec du citrate de sodium 10 mM.
